(19) Europäisches Patentamt
European Patent Office
Office européen des brevets

(11) Veröffentlichungsnummer: **0 252 393**
**B1**

(12) # EUROPÄISCHE PATENTSCHRIFT

(45) Veröffentlichungstag der Patentschrift: **16.01.91**

(51) Int. Cl.$^5$: **C 07 D 317/36,** D 06 M 13/00

(21) Anmeldenummer: **87109288.8**

(22) Anmeldetag: **27.06.87**

(54) **Neue polyfluorierte cyclische Carbonate, Verfahren zu ihrer Herstellung und ihre Verwendung.**

(30) Priorität: **04.07.86 DE 3622534**

(43) Veröffentlichungstag der Anmeldung:
**13.01.88 Patentblatt 88/02**

(45) Bekanntmachung des Hinweises auf die Patenterteilung:
**16.01.91 Patentblatt 91/03**

(84) Benannte Vertragsstaaten:
**BE CH DE FR GB IT LI NL**

(56) Entgegenhaltungen:
**TETRAHEDRON, Bnd. 40, Nr. 9, 1984, Seiten 1541-1544, Pergamon Press Ltd, Oxford, GB; S.BENEFICE et al.: "Réactivité des perfluoroiodoalcane avec les carbonates et pyrocarbonates d'alcoyles en présence de couple métallique zinc-cuivre"**

(73) Patentinhaber: **HOECHST AKTIENGESELLSCHAFT**
**Postfach 80 03 20**
**D-6230 Frankfurt am Main 80 (DE)**

(72) Erfinder: **Raab, Klaus, Dr.**
**Schneibsteinstrasse 6**
**D-8269 Burgkirchen (DE)**

**EP 0 252 393 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschriebung**

Die Erfindung betrifft neue polyfluorierte cyclische Carbonate, ein Verfahren zu ihrer Herstellung und ihre Verwendung.

Aus der US-Patentschrift 3,455,954 sind perfluorierte cyclische Carbonate der nachstehenden Formel bekannt, in der $R_f$ ein Perfluoralkylrest ist:

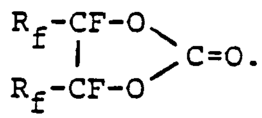

Diese vollständig fluorierten organischen Carbonate werden durch Fluorierung der entsprechenden H-haltigen cyclischen Carbonate erhalten.

In der Zeitschrift "Tetrahedron", Vol. 40, Nr. 9, Seiten 1541, bis 1544, 1984, wird die Umsetzung von $R_fZnI$ ($R_f$ = Perfluoralkyl) mit Ethylencarbonat gemäß nachstehender Reaktionsgleichung beschrieben, wobei unter anderem ein mit einem Perfluoralkylrest substituiertes Ethylencarbonat entsteht:

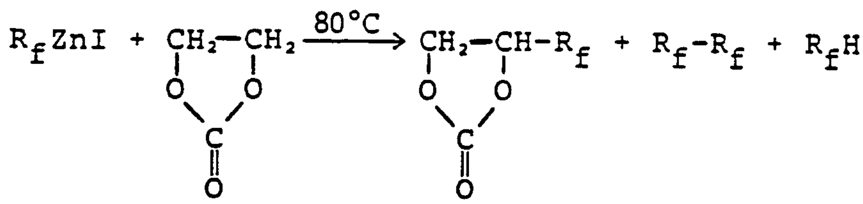


Das polyfluorierte cyclische Carbonat entsteht allerdings nur mit einer Ausbeute von etwa 5%, während die Verbindung $R_fH$ 90% des Umsetzungsproduktes ausmacht.

Der Erfindung liegt nun die Aufgabe zugrunde, neue polyfluorierte cyclische Carbonate bereitzustellen und ein Verfahren zu ihrer Herstellung zu schaffen, mit dem die neuen Verbindungen in einer relativ großen Ausbeute erhalten werden.

Die erfindungsgemäßen polyfluorierten cyclischen Carbonate entsprechen der nachstehenden Formel I,

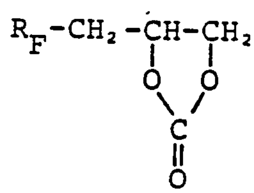

in der $R_F$ ein unverzweigter oder verzweigter Perfluoralkylrest mit 1 bis 18 C-Atomen, ein ω-Hydro oder ein ω-Halogen unverzweigter oder verzweigter Perfluoralkylrest mit 1 bis 18 C-Atomen oder ein Cycloperfluoralkylrest mit 4 bis 6 C-Atomen ist.

Das ω-Halogen ist vorzugsweise Brom oder Iod. Der unverzweigte (geradkettige) oder verzweigte Perfluoralkylrest und ebenso der ω-Hydro oder ω-Halogen Perfluoralkylrest enthält 1 bis 18 C-Atome, vorzugsweise 3 bis 14 und insbesondere 6 bis 12 C-Atome. Beispiele für solche Reste sind: $CF_3—$, $CF_3—CF_2—$, $(CF_3)_2CF—$, $CF_3—(CF_2)_3—$, $CF_3—(CF_2)_n—$, worin n 5, 6, 7, 8, 9, 11, 13 oder 15 ist, $(CF_3)_2CF—(CF_2)_3—$, $(CF_3CF_2CF_2)(CF_3)_2C—$, $(CF_3)_2CF—(CF_2)_4—$: $HCF_2—CF_2—$, $HCF_2—(CF_2)_3—$; $HalCF_2—CF_2—$, $HalCF_2—(CF_2)_3—$, $HalCF_2—(CF_2)_5—$ und $HalCF_2—(CF_2)_7—$, worin Hal Brom oder Iod ist. Der Cycloperfluoralkylrest ist vorzugsweise der Cycloperfluorpentylrest oder der Cycloperfluorhexylrest.

Die bevorzugte Bedeutung von $R_F$ in Formel I ist jene eines unverzweigten oder verzweigten Perfluoralkylrestes, wobei die unverzweigten Perfluoralkylreste besonders bevorzugt sind.

Die erfindungsgemäßen polyfluorierten cyclischen Carbonate sind in der Regel bei Raumtemperatur farblose, feste und kristalline Stoffe.

Das erfindungsgemäße Verfahren zur Herstellung der neuen polyfluorierten cyclischen Carbonate der Formel I ist dadurch gekennzeichnet, daß ein polyfluoriertes Halogenhydrin der nachstehenden Formel II

$$R_F—CH_2—CH(Hal)—CH_2OH,$$

in der $R_F$ die obengenannte Bedeutung hat und Hal für ein Halogen steht, vorzugsweise Brom oder Iod, mit einem Alkalimetallcarbonat, Alkalimetallhydrogencarbonat oder einem Alkylammoniumhydrogencarbonat im Molverhältnis von 1:1 bis 2 in Gegenwart eines aprotischen Lösungsmittels umgesetzt wird, mit der Maßgabe, daß bei Einsatz von Alkalimetallcarbonat oder -hydrogencarbonat die Umsetzung bei einer Temperatur von 25 bis 100°C, vorzugsweise 40 bis 80°C, und bei Einsatz von Alkylammoniumhydrogencarbonat die Umsetzung bei einer Temperatur von −20 bis 40°C, vorzugsweise −10 bis 20°C, durchgeführt wird, und aus dem Umsetzungsprodukt die angestrebte Verbindung (das ist die Verbindung gemäß Formel I) isoliert wird.

Der Herstellung der erfindungsgemäßen Verbindungen liegt — beispielsweise im Falle von Natriumhydrogencarbonat als zweiter Reaktionskomponente — die nachstehende Reaktionsgleichung zugrunde:

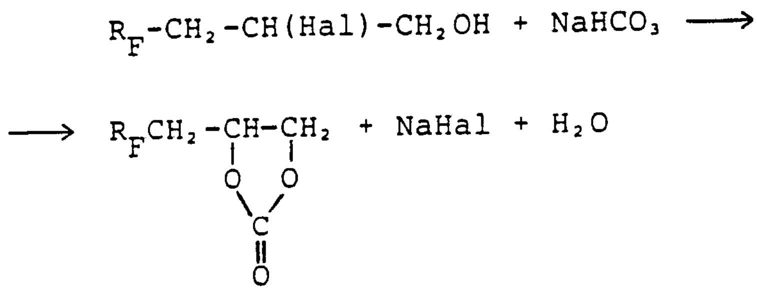

worin $R_F$ und Hal die obengenannte Bedeutung haben.

Die eine der beiden Ausgangsverbindungen (1. Reaktionskomponente) ist also ein polyfluoriertes Halogenhydrin der obigen Formel II, in der Hal ein Halogen ist, vorzugsweise Brom oder Iod, und für $R_F$ das gilt, was über $R_F$ der Formel I gesagt worden ist. Beim eingesetzten Halogenhydrin kann es sich auch um eine Mischung von verschiedenen $R_F$-Resten handeln, vorzugsweise von verschiedenen Perfluoralkylresten. Die andere Ausgangsverbindung (2. Reaktionskomponente) ist eine der oben angegebenen Carbonat- oder Hydrogencarbonat-Verbindungen. Das Alkalimetall in diesen Verbindungen ist vorzugsweise Kalium oder Natrium, wobei Natrium besonders bevorzugt ist. Der alkylrest in den Alkylammoniumhydrogencarbonaten ist vorzugsweise eine $C_1$ bis $C_4$-Alkylgruppe und insbesondere Methyl oder Ethyl. Von den Alkylammoniumhydrogencarbonaten sind die Tetraalkylammoniumhydrogencarbonate bevorzugt.

Die beim erfindungsgemäßen Verfahren einzusetzenden beiden Ausgangsverbindungen sind schon seit langem bekannt.

Nach der oben angegebenen Reaktionsgleichung sind die beiden Ausgangsverbindungen im molaren Verhältnis von 1:1 einzusetzen. Es hat sich als zweckmäßig erwiesen, von der 2. Reaktionskomponente, der Carbonat- oder Hydrogencarbonat-Verbindung, einen Überschuß einzusetzen. Das Molverhältnis zwischen dem polyfluorierten Halogenhydrin und dem Carbonat oder Hydrogencarbonat liegt demnach bei 1:1 bis 2, vorzugsweise bei 1:1 bis 1,5.

Die erfindungsgemäße Umsetzung wird in Gegenwart eines aprotischen Lösungsmittels durchgeführt. Geeignete aprotische Lösungsmittel sind Dimethylformamid, Dimethylacetamid, N-Methylpyrrolidon, Dimethylsulfoxid, Sulfolan, Tetramethylharnstoff, Hexamethylphosphorsäuretriamid, Acetonitril, Monoethylenglykoldimethylether oder Diethylenglykoldimethylether.

Es hat sich herausgestellt, daß bei Einsatz von Alkalimetallcarbonat und Alkalimetallhydrogencarbonat als der 2. Reaktionskomponente Dimethylformamid und N-Methylpyrrolidon besonders geeignete Lösungsmittel sind, und bei Einsatz von Alkylammoniumhydrogencarbonat Acetonitril das besonders geeignete Lösungsmittel ist. Die Menge an Lösungsmittel, in dem die polyfluorierten Halogenhydrine im wesentlichen löslich und die Carbonate und Hydrogencarbonate fast unlöslich sind, kann in weiten Grenzen variieren. Aus Zweckmäßigkeitsgründen wird soviel Lösungsmittel genommen, daß aus eingesetztem Halogenhydrin, Carbonat oder Hydrogencarbonat und Lösungsmittel eine gut rührbare Suspension vorliegt. In der Regel werden 0,1 bis 15 l Lösungsmittel, vorzugsweise 0,3 bis 6 l, pro mol polyfluoriertes Halogenhydrin eingesetzt.

Was die Reaktionstemperatur beim erfindungsgemäßen Verfahren betrifft, had sich herausgestellt, daß diese je nach Art der 2. Reaktionskomponente in einem anderen Bereich liegt und insbesondere einen bestimmten Maximalwert nicht überschreiten soll. Bei Einsatz von Alkalimetallcarbonat oder Alkalimetallhydrogencarbonat als 2. Reaktionskomponente soll die Umsetzungstemperatur nicht über etwa 100°C liegen. Bei einer Temperatur von etwa über 100°C tritt nämlich eine mit steigender Temperatur relativ schnell zunehmende Zersetzung und/oder Umwandlung der gebildeten und angestrebten Verbindungen ein. Die Reaktionstemperatur im Falle von Alkalimetallcarbonat und Alkalimetallhydrogencarbonat als 2. Reaktionskomponente liegt demnach bei 25 bis 100°C, vorzugsweise bei 40 bis 80°C. Temperaturen unter etwa 25°C sind zwar möglich, die Reaktionszeiten werden aber unverhältnismäßig lang. Bei Einsatz von Alkylammoniumhydrogencarbonat als 2. Reaktionskomponente sol die Umsetzungstemperatur nicht über etwa 40°C liegen, da bei höheren Temperaturen, ebenso wie im Falle der Carbonat- und Hydrogencarbonat-Verbindungen, Zersetzung und/oder Umwandlung der gebildeten und angestrebten Verbindungen eintritt. Die Reaktionstemperatur liegt demnach bei −20°C bis +40°C, vorzugsweise −10°C bis +20°C. Bei einer Temperatur von unter −20°C ist die Reaktionszeit bereits unverhältnismäßig lang.

Die Reaktionszeit bei der erfindungsgemäßen Umsetzung, die im wesentlichen von der Reaktionstemperatur abhängt, liegt im Bereich von 1 bis 30 h.

Die erfindungsgemäße Umsetzung wird in der Regel in der Weise durchgeführt, daß die Ausgangsverbindungen und das Lösungsmittel in einem Reaktionsgefäß eingebracht werden und die erhaltene Suspension unter Rühren auf Reaktionstemperatur gebracht wird, worauf die Umsetzung abläuft. Im Falle von Reaktionstemperaturen, die unterhalb Raumtemperatur liegen, wird eine entsprechende Abkühlung der Ausgangsverbindungen, des Lösungsmittel und der Suspension vorgenommen. Der Verlauf und das Ende der Umsetzung kann zum Beispiel NMR-spektroskopisch verfolgt werden. Es hat sich als zweckmäßig erwiesen, im Reaktionsgefäß eine Kohlendioxidatmosphäre aufrechtzuhalten. Dies kann zum Beispiel dadurch erreicht werden, daß ein geringer Kohlendioxidstrom kontinuierlich oder diskontinuierlich durch die Suspension oder den freien Raum des Reaktionsgefäßes geblasen wird.

Zur Isolierung der angestrebten erfindungsgemäßen Verbindungen nach Beendigung der Umsetzung wird das Umsetzungsprodukt von den nicht angestrebten Verbindungen, wie anorganischen oder organischen Salzen, zum Beispiel NaI und $N(CH_3)_4I$, überschüssigem Carbonat oder Hydrogencarbonat sowie destillierbaren Nebenprodukten befreit, zum Beispiel durch Filtration beziehungsweise Destillation, und das erhaltene Filtrat beziehungsweise der Destillationsrückstand oder das Destillat mit Wasser versetzt, worauf die angestrebte Verbindung ausfällt und abgetrennt wird. Die erhaltenen erfindungsgemäßen Verbindungen können zur Reinigung mit Wasser gewaschen und ein- oder mehrmals umkristallisiert werden.

Die erfindungsgemäßen polyfluorierten cyclischen Carbonate der allgemeinen Formel I eignen sich zur oleophoben und/oder hydrophoben Ausrüstung von Textilmaterialien. Sie eignen sich ferner als oberflächenaktives Hilfsmittel, beispielsweise als Zusatz zu Wachsen, Fetten und Ölen. Die erfindungsgemäßen Carbonate stellen ferner Produkte zur Herstellung von wertvollen Fluorverbindungen oder Fluorpolymeren dar. So reagieren sie in Gegenwart katalytischer Mengen von Basen, vorzugsweise in Gegenwart einer katalytischen Menge von Alkalimetallhydroxiden, wie KOH, NaOH, Alkalimetallcarbonaten, wie $K_2CO_3$, $Na_2CO_3$, Alkalimetallhydrogencarbonaten, wie $KHCO_3$, $NaHCO_3$, Tetraalkylammoniumhydrogencarbonaten, wie Tetramethylammoniumhydrogencarbonat und tertiäre Amine, wie Triethylamin, zu (Perfluoralkyl)propenolen der Formel $R_F—CH=CH—CH_2OH$, worin $R_F$ die obengenannte Bedeutung hat. Diese Reaktion verläuft in guter Ausbeute. Sie stellt einen vorteilhaften Weg dar zur Herstellung der genannten Propenole, die begehrte Ausgangsverbindungen zur Herstellung von teilfluorierten Polyacrylaten sind, die gute Hydrophobierungs- und Oleophobierungsmittel darstellen. Die erfindungsgemäßen Carbonate reagieren ferner unter Basenkatalyse mit Wasser zu (Perfluoralkyl)-propandiolen der Formel $R_F—CH_2—CH(OH)—CH_2OH$, worin $R_F$ die obengenannte Bedeutung hat; dies ist ein ganz einfacher Weg zur Herstellung von fluorierten Diolen. Die erfindungsgemäßen Carbonate stellen andererseits geeignete Comonomere zur Modifizierung von Polymeren dar, beispielsweise zur Modifizierung von Ethylencarbonat/Terephthalsäure-Copolymeren.

Von den genannten Verwendungen der erfindungsgemäßen Verbindungen sind die Herstellung der genannten (Perfluoralkyl)propenolen und die Oleophobierung und/oder Hydrophobierung von Textilien besonders bedeutungsvoll. Als Textilien kommen insbesondere solche aus nativer und/oder regenerierter Cellulose, wie Baumwolle, Leinen, Zellwolle und Celluloseacetat, und solche aus Wolle in Betracht. Es können auch Textilien aus zum Beispiel Polyamiden, Polyestern, Polyacrylnitril oder Polypropylen, oder Textilien aus Mischungen der genannten Produkte eingesetzt werden. Das Textilmaterial kann beispielsweise als Faser, Faden oder Gewebe vorliegen. Die Applikation der Verbindungen wird nach den üblichen Arbeitsweisen durchgeführt, beispielsweise durch Tauchen in entsprechende Lösungsmittelflotten, durch Foulardieren, Sprühen oder Klotzen. Die erfindungsgemäßen Verbindungen werden in einer Menge von 0,05 bis 10 Gew.-%, vorzugsweise 0,1 bis 5 Gew.-%, bezogen auf das Gewicht des Textilmaterials, appliziert. Sie verleihen den Textilmaterialien eine ausgezeichnete Hydrophobie und Oleophobie.

Die Erfindung wird nun an Beispielen noch näher erläutert.

### Beispiel 1

Es wird die erfindungsgemäße Verbindung der nachstehenden Formel Ia hergestellt:

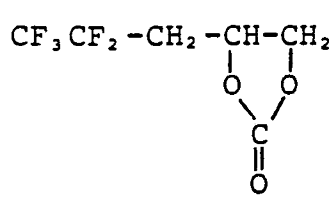

In einem 1-l-Dreihalskolben mit Rückflußkühler, Rührer und Innenthermometer werden 17,6 g (0,21 mol) $NaHCO_3$, 60,8 g (0,20 mol) $CF_3CF_2—CH_2—CHI—CH_2OH$ und 350 ml Dimethylformamid eingebracht. Die Suspension wird unter Rühren auf 80°C erhitzt und bei dieser Temperatur unter Rühren 16 h lang gehalten, worauf die Umsetzung beendet ist. Der Kolbeninhalt wird auf Raumtemperatur abgekühlt und filtriert. Das Filtrat wird mit etwa 0,1 l Wasser versetzt, der ausgefallene Niederschlag abgenutscht, mit Wasser gewaschen und im Exsiccator über $P_4O_{10}$ im Ölpumpenvakuum getrocknet. Das so erhaltene Produkt entsprechend der obigen Formel Ia ist farblos bis blaßgelb und hat einen Schmelzpunkt von 83°C

und einen Siedepunkt von 130 bis 135°C bei 20 mbar. Die Ausbeute beträgt 63 Gew.-% der Theorie. Nach Reinigung des Produktes durch Umkristallisieren aus $CCl_4$ beträgt sein Schmelzpunkt 84°C.

Beispiel 2

Es wird die erfindungsgemäße Verbindung der nachstehenden Formel Ib hergestellt:

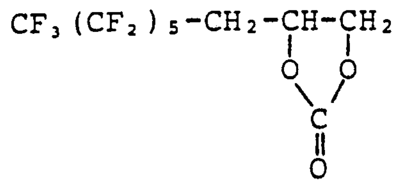

In einem mit $CaCl_2$-Rohr, Rühren und Innenthermometer ausgestatteten 1-l-Dreihalskolben werden — jeweils auf −7°C abgekühlt — 100,8 g (0,20 mol) $C_6F_{13}$—$CH_2$—CHI—$CH_2OH$, 28,4 g (0,21 mol) $N(CH_3)_4HCO_3$ und 900 ml wasserfreies Acetonitril eingebracht, worauf die Suspension unter Rühren auf −5°C gebracht wird. Nach Durchleiten eines geringen $CO_2$-Stromes durch die Suspension 10 min lang zur Erzeugung einer $CO_2$-Atmosphäre wird die Suspension unter weiterem Rühren bei −5°C 20 h lang gehalten, worauf die Umsetzung beendet ist. Der Kolbeninhalt wird auf Raumtemperatur gebracht und abgesaugt (Abtrennung vom schwerlöslichen $N(CH_3)_4I$). Das Filtrat wird mit etwa 1 l Wasser versetzt, der ausgefallene Niederschlag wird abgenutscht, mit Wasser und anschließend mit Diethylether zur Entfernung evtl. vorhandener organischer Nebenprodukte gewaschen und im Vakuum getrocknet. Das so erhaltene Produkt entsprechend der obigen Formel Ib ist farblos und hat einen Schmelzpunkt von 118°C (Umkristallisierung aus $CHCl_3$). Die Ausbeute beträgt 82 Gew.-% der Theorie.

Beispiel 3

Es wird die erfindungsgemäße Verbindung der nachstehenden Formel Ic hergestellt:

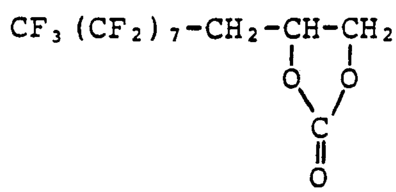

In einem mit Rückflußkühler und $CaCl_2$-Rohr, Rührer und Innenthermometer ausgetatteten 4-l-Dreihalskolben werden 28,4 g (0,21 mol) $N(CH_3)_4HCO_3$, 120,8 g (0,20 mol) $C_8F_{17}$—$CH_2$—CHI—$CH_2OH$ und 700 ml Acetonitril eingebracht, Die Suspension wird auf 10°C abgekühlt, und 10 min lang wird ein geringer $CO_2$-Strom durch die Suspension geleitet. Nun wird die Suspension bei 8 bis 10°C 12 Stunden lang unter Rühren gehalten, worauf die Umsetzung beendet ist. Der Kolbeninhalt wird mit 2 l Wasser versetzt. Nach kurzem Rühren läßt man den farblosen Niederschlag, bestehend im wesentlichen aus $N(CH_3)_4I$ und der angestrebten erfindungsgemäßen Verbindung absetzen. Die überstehende Lösung wird dekantiert und filtriert. Der Niederschlag wird zur Abtrennung des $N(CH_3)_4I$ mehrmals mit Wasser und anschließend mit Acetonitril gewaschen und hierauf im Vakuum getrocknet. Das so erhaltene Produkt entsprechend der obigen Formel Ic ist farblos und hat einen Schmelzpunkt von 136°C (Umkristallisierung aus $CHCl_3$). Die Ausbeute beträgt 74 Gew.-% der Theorie.

Beispiel 4

Es wird die erfindungsgemäße Verbindung der nachstehenden Formel Id hergestellt:

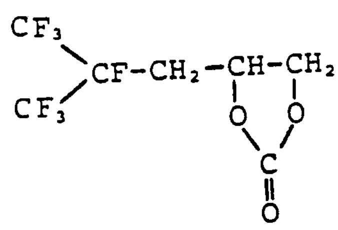

In einem 1-l-Dreihalskolben mit Rückflußkühler, Rührer und Innenthermometer werden 29,4 g (0,35 mol) $NaHCO_3$, 88,5 g (0,25 mol) $(CF_3)_2CF$—$CH_2$—CHI—$CH_2OH$ und 400 ml Dimethylformamid eingebracht. Die Suspension wird unter Rühren auf 80°C erhitzt und bei dieser Temperatur unter Rühren 18 h lang gehalten, worauf die Umsetzung beendet ist. Der Kolbeninhalt wird auf Raumtemperatur abgekühlt und filtriert. Das Filtrat wird mit 0,7 l Wasser versetzt. Die flüssige Unterphase wird im Scheidetrichter

abgetrennt, mit Wasser ausgeschüttelt und im Wasserstrahlpumpenvakuum destilliert. Das so erhaltene Produkt entsprechend der obigen Formel Id ist farblos und hat einen Siedepunkt von 117—120°C bei 10 mbar. Sein Schmelzpunkt liegt bei 26°C. Die Ausbeute beträgt 52 Gew.-% der Theorie.

Beispiel 5

Es wird die erfindungsgemäße Verbindung der nachstehenden Formel Ie hergestellt:

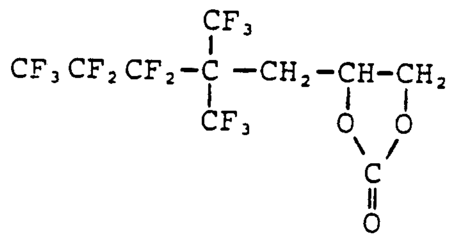

In einem 500-ml-Dreihalskolben mit Rückflußkühler, Rührer und Innenthermometer werden 4,4 g (52 mmol) $NaHCO_3$, 22,9 g (50 mmol) $CF_3CF_2CF_2(CF_3)_2C—CH_2—CHBr—CH_2OH$ und 150 ml Dimethylformamide eingebracht. Die Suspension wird unter Rühren auf 80°C erhitzt und bei dieser Temperatur unter Rühren 20 h lang gehalten, worauf die Umsetzung beendet ist. Der Kolbeninhalt wird auf Raumtemperatur abgekühlt und filtriert. Das Filtrat wird mit etwa 0,2 l Wasser versetzt. Die flüssige Unterphase wird im Scheidetrichter abgetrennt. Nach weiterem Waschen mit Wasser wird die flüssige Unterphase fest. Dieser Feststoff wird abgesaugt, mit Wasser und anschließend mit wenig Diethylether gewaschen und hierauf im Vakuum getrocknet. Das so erhaltene Produkt entsprechend der obigen Formel Ie ist farblos und hat einen Schmelzpunkt von 58°C. Die Ausbeute beträgt 66 Gew.-% der Theorie.

Beispiel 6

Es wird der erfindungsgemäße Verbindung des Beispiels 2, das ist die Verbindung der Formel Ib, hergestellt. In einem 500-ml-Dreihalskolben mit Rückflußkühler, Rührer und Innenthermometer werden 15,9 g (0,15 mol) $Na_2CO_3$, 50,4 g (0,10 mol) $CF_3(CF_2)_5—CH_2—CHI—CH_2OH$ und 300 ml Dimethylformamid eingebracht. Die Suspension wird unter Rühren auf 80°C erhitzt und bei dieser Temperatur unter Rühren 16 h lang gehalten. Der Kolbeninhalt wird auf Raumtemperatur abgekühlt und filtriert. Das Filtrat wird mit 0,5 l Wasser versetzt. Der ausgefallene Niederschlag wird abgenutscht, mit Wasser und anschließend mit Diethylether gewaschen und im Vakuum getrocknet. Das so erhaltene Produkt entsprechend der Formel Ib hat die im Beispiel 2 angegebenen Eigenschaften. Die Ausbeute beträgt 61 Gew.-% der Theorie.

Beispiel 7

Beispiel 6 wird wiederholt mit folgenden Abänderungen:

Statt 15,9 g $Na_2CO_3$ werden 12,6 g (0,15 mol) $NaHCO_3$ eingesetzt;

statt 300 ml Dimethylformamid werden 300 ml N-Methylpyrrolidon eingesetzt,

die Reaktionstemperatur wird bei 70°C gehalten.

Die Ausbeute an der erfindungsgemäßen Verbindung der Formel Ib beträgt 49 Gew.-% der Theorie.

(Aus der Diethylether-Waschlösung können 39 Gew.-% von unumgesetztem $CF_3(CF_2)_5—CH_2—CH_2—CH_2OH$, bezogen auf eingesetztes $CF_3(CF_2)_5—CH_2—CHI—CH_2OH$, isoliert werden.)

Beispiel 8

Beispiel 6 wird wiederholt mit folgenden Abänderungen: Statt 15,9 g $Na_2CO_3$ werden 11 g (0,11 mol) $KHCO_3$ eingesetzt;

die Reaktionstemperatur wird bei 55 is 60°C gehalten;

die Reaktionsdauer wird auf 8 h begrenzt.

Die Ausbeute an der erfindungsgemäßen Verbindung der Formel Ib beträgt 44 Gew.-% der Theorie.

Die Konstitution der in den Beispielen hergestellten erfindungsgemäßen polyfluorierten cyclischen Carbonate wurde durch $^1H$—NMR-, $^{13}C$—NMR-, $^{19}F$—NMR-Skektroskopie, IR-Spektroskopie und Elementaranalyse bestimmt.

In den folgenden Beispielen 9 und 10 wird die Umsetzung der erfindungsgemäßen polyfluorierten cyclischen Carbonate zu den wertvollen (Perfluoralkyl)propenolen näher beschrieben.

Beispiel 9

Umsetzung des erfindungsgemäßen Carbonates des Beispiels 2 zu dem Perfluoralkylpropenol der Formel $CF_3(CF_2)_5—CH=CH—CH_2OH$:

In einem 500-ml-Dreihalskolben mit Rückflußkühler, Rührer und Thermometer werden 42,0 g (0,1 mol) des erfindungsgemäßen Carbonates der Formel Ib (vgl. Beispiel 2), 1,0 g (0,01 mol) $KHCO_3$ und 200 ml Dimethylacetamid eingebracht. Die Mischung wird bei 90°C 12 h lang gerührt, bis die $CO_2$-Entwicklung aufgehört hat. Der Kolbeninhalt wird auf Raumtemperatur abgekühlt und mit 0,5 l Wasser versetzt. Die flüssige Unterphase wird abgetrennt, mehrmals mit Wasser ausgerührt und destilliert (Siedepunkt: 83 bis

85°C bei ca. 10 mbar). Das Destillat besteht aus $CF_3(CF_2)_5$—CH=CH—$CH_2OH$. Die Ausbeute beträgt 92 Gew.-% der Theorie.

Beispiel 10

Umsetzung wie im Beispiel 9, wobei anstelle von $KHCO_3$, $NaHCO_3$ eingesetzt und das Dimethylacetamid weggelassen wird. In einem 100-ml-Kolben mit Rückflußkühler und Magnetrührer werden 21,0 g (50 mmol) des erfindungsgemäßen Carbonates und 0,25 g (3 mmol) $NaHCO_3$ eingebracht. Die Mischung wird auf 150°C erhitzt und die entstehende Schmelze 6 h lang gerührt, bis die $CO_2$-Entwicklung aufgehört hat. Der Kolbeninhalt wird danach im Vakuum destilliert. Die Ausbeute an $CF_3(CF_2)_5$—CH=CH—$CH_2OH$ beträgt 81 Gew.-% der Theorie.

**Patentansprüche**

1. Polyfluorierte cyclische Carbonate der nachstehenden Formel I

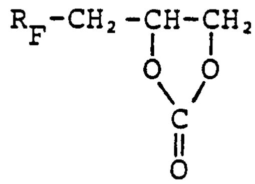

in der $R_F$ ein unverzweigter oder verzweigter Perfluoralkylrest mit 1 bis 18 C-Atomen, ein ω-Hydro oder ein ω-Halogen unverzweigter oder verzweigter Perfluoralkylrest mit 1 bis 18 C-Atomen oder ein Cycloperfluoralkylrest mit 4 bis 6 C-Atomen ist.

2. Polyfluorierte cyclische Carbonate nach Anspruch 1, wobei $R_F$ ein unverzweigter oder verzweigter Perfluoralkylrest mit 3 bis 14 C-Atomen ist.

3. Polyfluorierte cyclische Carbonate nach Anspruch 1, wobei $R_F$ ein unverzweigter Perfluoralkylrest mit 3 bis 14 C-Atomen ist.

4. Verfahren zur Herstellung von polyfluorierten cyclischen Carbonaten nach Anspruch 1, dadurch gekennzeichnet, daß ein polyfluoriertes Halogenhydrin der nachstehenden Formel II

$$R_F—CH_2—CH(Hal)—CH_2OH,$$

in der $R_F$ die obengenannte Bedeutung hat und Hal für ein Halogen steht, mit einem Alkalimetallcarbonat, Alkalimetallhydrogencarbonat oder einem Alkylammoniumhydrogencarbonat im Molverhältnis von 1:1 bis 2 in Gegenwart eines aprotischen Lösungsmittels umgesetzt wird, mit der Maßgabe, daß bei Einsatz von Alkalimetallcarbonat oder Alkalimetallhydrogencarbonat die Umsetzung bei einer Temperatur von 25 bis 100°C und bei Einsatz von Alkylammoniumhydrogencarbonat die Umsetzung bei einer Temperatur von −20 bis 40°C durchgeführt wird, und aus dem Umsetzungsprodukt die angestrebte Verbindung isoliert wird.

5. Verwendung der polyfluorierten cyclischen Carbonate nach Anspruch 1 zur hydrophoben und oleophoben Ausrüstung von Textilien.

**Revendications**

1. Carbonates cycliques polyfluorés ayant la formule I ci-après

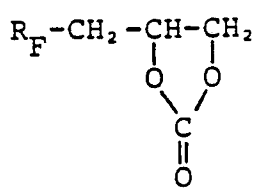

dans laquelle $R_F$ est un radical perfluoralkyle ramifié ou non ramifié ayant de 1 à 18 atomes de carbone, un radical ω-hydrogéno- ou ω-halogéno-perfluoralkyle ramifié ou non ramifié ayant de 1 à 18 atomes de carbone, ou encore un radical cycloperfluoralkyle ayant de 4 à 6 atomes de carbone.

2. Carbonates cycliques polyfluorés selon la revendication 1, où $R_F$ est un radical perfluoralkyle à chaîne droite ou ramifiée ayant de 3 à 14 atomes de carbone.

3. Carbonates cycliques polyfluorés selon la revendication 1, où $R_F$ est un radical perfluoralkyle à chaîne droite ayant de 3 à 14 atomes de carbone.

4. Procédé de préparation de carbonates cycliques polyfluorés selon la revendication 1, caractérisé en ce qu'on fait réagir une halogénhydrine polyfluorée ayant la formule II ci-après

$$R_F—CH_2—CH(Hal)—CH_2OH,$$

dans laquelle $R_F$ a les significations données ci-dessus et Hal représente un halogène,

avec un carbonate de métal alcalin, un hydrogénocarbonate de métal alcalin ou un hydrogénocarbonate d'alkylammonium, selon une proportion molaire de 1:1 à 2, en présence d'un solvant aprotique, à la condition que, quand on utilise un carbonate ou un hydrogénocarbonate de métal alcalin, la réaction soit mise en oeuvre à une température de 25 à 100°C et que, quand on utilise de l'hydrogénocarbonate d'alkylammonium, la réaction soit mise en oeuvre à une température de −20 à 40°C, et que l'on isole du produit de réaction le composé recherché.

5. Utilisation des carbonates cycliques polyfluorés selon la revendication 1 pour l'apprêt hydrophobe et oléophobe de textiles.

## Claims

1. Polyfluorinated cyclic carbonates of the formula I below

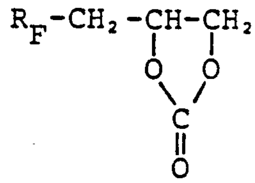

in which $R_F$ is an unbranched or branched perfluoroalkyl radical having 1 to 18 carbon atoms, an unbranched or branched ω-hydro- or ω-haloperfluoroalkyl radical having 1 to 18 carbon atoms, or a cycloperfluoroalkyl radical having 4 to 6 carbon atoms.

2. Polyfluorinated cyclic carbonates as claimed in claim 1, where $R_F$ is an unbranched or branched perfluoroalkyl radical having 3 to 14 carbon atoms.

3. Polyfluorinated cyclic carbonates as claimed in claim 1, where $R_F$ is an unbranched perfluoroalkyl radical having 3 to 14 carbon atoms.

4. A process for the preparation of polyfluorinated cyclic carbonates as claimed in claim 1, characterised in that a polyfluorinated halohydrin of the following formula II

$$R_F—CH_2—CH(Hal)—CH_2OH$$

in which $R_F$ has the above-mentioned meaning and Hal represents a halogen, is reacted with an alkali metal carbonate, an alkali metal hydrogen carbonate or an alkylammonium hydrogen carbonate in the molar ratio 1:1 to 2 in the presence of an aprotic solvent, with the proviso that, when an alkali metal carbonate or alkali metal hydrogen carbonate is used, the reaction is carried out at a temperature of 25 to 100°C, and when an alkylammonium hydrogen carbonate is used, the reaction is carried out at a temperature of −20 to 40°C, and the intended compound is isolated from the reaction product.

5. The use of the polyfluorinated cyclic carbonates as claimed in claim 1 for waterproof and oilproof finishing of textiles.